# EUROPEAN PATENT APPLICATION

(11) **EP 2 520 284 A1**
(43) Date of publication of application: **07.11.2012**
(21) Application number: 12178119.9
(22) Date of filing: 16.09.2008
(51) Int. Cl.: A61K 9/14, A61K 9/16, A61K 9/48, A61K 9/58, A61P 19/02, A61K 31/192, A61P 29/00

(54) **Pharmaceutical compositions of rhein or diacerein**

(30) Priority: 27.09.2007 IN MU18992007; 27.09.2007 IN MU18982007; 27.09.2007 IN MU19002007; 27.09.2007 IN MU19012007; 24.03.2008 IN MU05772008; 24.03.2008 IN MU05782008; 24.03.2008 IN MU05792008; 24.03.2008 IN MU05802008; 26.05.2008 IN MU11142008; 18.07.2008 IN MU15192008; 18.07.2008 IN MU15202008
(62) Divisional of application: 08807676.5
(71) Applicant: Wockhardt Limited, Aurangabad 431210 (IN)
(72) Inventor: Dabre, Rahul, 440015 Nagpur (IN); Jain, Girish, Kumar, 10034 Delhi (IN); Sandal, Roshanlal, 142050 Ferozpur (IN); Thakkar, Vikrant, 131001 Sampat (IN)
(74) Representative: May, Mark Andrew

(57) **Abstract**

The invention relates to pharmaceutical compositions of rhein or diacerein, or salts or esters or prodrugs thereof which are bioequivalent to a 50 mg diacerein formulation marketed under the trade name Art 50^{®}, The compositions exhibit no variability in fed and fasted state conditions. The compositions also result in significant reduction in side effects such as, soft stools as compared to Art 50^{®}. The invention also relates to the methods for preparing such compositions.

## Description

### Field of the Invention

The invention relates to pharmaceutical compositions of rhein or diacerein, or salts or esters or prodrugs thereof which are bioequivalent to a 50 mg diacerein formulation marketed under the trade name Art 50^{®}. The compositions exhibit no variability in fed and fasted state conditions. The compositions also result in significant reduction in side effects such as, soft stools as compared to Art 50^{®}. The invention also relates to methods for preparing such compositions.

### Background of the Invention

Chemically, rhein is 9,10-dihydro-4, 5-dihydroxy-9, 10-dioxo-2-anthracene carboxylic acid having a structure of Formula I and diacerein is 4,5-bis (acetyloxy) 9,10-dihydro-4, 5-dihydroxy-9, 10-dioxo-2-anthracenecarboxylic acid having a structure of Formula II. Diacerein is widely used in the treatment of osteoarthritis and has a unique mode of action that differentiates it from non-steroidal anti-inflammatory drugs (NSAIDs) and other conventional forms of drug therapy. Presently, diacerein capsules are available in 50 mg strength and are marketed by Negma in France and under the trade name Art 50^{®}.

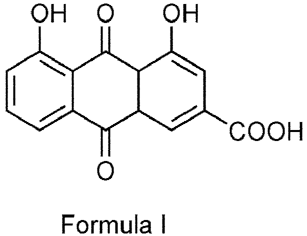

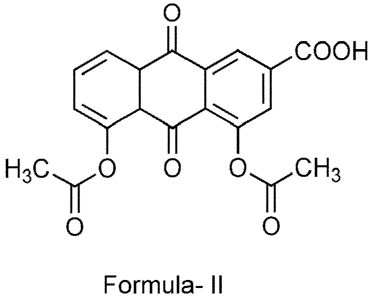

Diacerein is practically insoluble in solvents such as water, alcohols, acetone, dichloromethane and chloroform, which are generally used in pharmaceutical preparations. Although diacerein can be administered by oral route but it cannot be completely absorbed by the digestive tract, and this incomplete absorption may result in undesirable side effects such as, soft stools.

In order to overcome these problems, various derivatives, pharmaceutical compositions and specific galenic forms have been proposed in the literature. For example, European patent, EP 243,968 discloses a potassium salt of diacerein, which is water-soluble and can be used in the preparation of compositions for parenteral administration.

European Patent No EP904060 discloses pharmaceutical compositions of rhein or diacerein, wherein rhein or diacerein is co-micronized with sodium lauryl sulfate.

European Patent Nos. EP263083; 264989 and 446753 disclose controlled release or delayed release compositions like multiplicity of pellets coated with drug and coating membrane or granules of drug coated with polymers or loading polymeric particles of water swellable cross-linked polymer with drug.

U.S. Patent Nos. 5,225,192 and 5,569,469 describe different poorly soluble medicaments supported on polymeric particles of water swellable cross-linked polymer with drug.

U.S. Patent No. 5,952,383 and European Patent No. EP 862423B1 describe pharmaceutical compositions of diacerein, rhein and their salts along with liquid support systems like oils, suspending agents, homogenizing agents and other excipients.

It is known that when 50 mg diacerein formulation currently marketed under the trade name Art 50^{®} is given orally in fasted conditions, due to fast gastric emptying, most of the diacerein remains unabsorbed and unabsorbed diacerein gets converted into rhein before reaching colon. At the colonic site, the rhein degrades to rhein-9-anthrone which causes significant soft stool effect. This soft stool effect is observed in about 50% of the patients after first few doses of Art 50^{®}. In fact, about 30-40% soft stool effect is expected due to inherent pharmacokinetic property of diacerein, i.e diacerein undergoes enterohepatic circulation, wherein rhein gets conjugated in liver to form rhein-glucuronide, which on reaching colon gets converted to rhein-9-anthrone and hence causes soft stool effect.

On the other hand, when Art 50^{®} is given in fed conditions, the gastric emptying is delayed in the presence of food. The longer residence time in upper part of the gastrointestinal tract accompanied with gastric fluids results in increased absorption of diacerein. This increase in absorption is up to 25% leading to comparatively less amount of unabsorbed diacerein to reach colon and hence reduction in soft stools. However, this reduction in soft stools is not significant. It was also observed that when the diacerein formulation described in EP 904060 comprising co-micronized diacerein with sodium lauryl sulfate is given, it results in only about 18% reduction in soft stools, which is not significant. This reduction in soft stools is not due to dose reduction but it is related to increased absorption of the diacerein leading to lesser amount of unabsorbed diacerein reaching colon. The diacerein formulation described in EP 904060 also exhibits drastic variation in both fed and fasted conditions. So, prior art formulations are discriminatory with respect to both fast and fed conditions. Additionally, prior art formulations are also eclipsed with undesirable soft stool effect.

Due to soft stool effect, prior art formulations (Art 50^{®} and Art 40) are initially given once a day for about two months, so that the patient's gastrointestinal tract gets acclimatized to the side effect of diacerein. After that, the dosage regimen is scheduled for twice a day for both Art 50^{®} and Art 40. Although, this adjustment of the dosage regimen improves patient's compliance to some extent, but there is no reduction in side effects. There still exists a need to develop new formulations or compositions which are likely to achieve a higher rate and extent of absorption of diacerein leading to improved bioavailability and at the same time shows significant reduction in side effects such as, soft stools.

In spite of the attempts in the prior art, described above, the inventors are not aware of successful attempts to improve the absorption of diacerein and significant reduction in soft stools. As described below, the inventors have surprisingly found that compositions of the invention result in a higher rate and extent of absorption from the gastrointestinal tract and significant reduction (at least 25%) in soft stools. The inventors also have surprisingly found that the compositions can be given with or without food without affecting the rate and extent of absorption. The inventors have further noticed that there is no need to co-micronize diacerein with any surfactant to get a formulation, which is bioequivalent to the commercially available diacerein 50 mg solid oral dosage form (Art 50^{®}).

Thus, the compositions of the invention, overcome all the commonly encountered problems exemplified in the prior art. When the compositions of the invention are given orally, diacerein gets completely absorbed in upper part of intestine and there remains no unabsorbed diacerein reaching colon, resulting in a significant reduction in soft stools effect from about 60-70%. Furthermore, the compositions of the invention are bioequivalent to 50 mg diacerein formulation currently marketed under the trade name Art 50^{®} showing no variability whether administered in fed or fasted state conditions.

### Summary of the Invention

In one general aspect there is provided a melt granulated pharmaceutical composition comprising rhein or diacerein, or salts or esters or prodrugs thereof.

In another general aspect there is provided a pharmaceutical composition comprising from about 20mg to about 45 mg of rhein or diacerein, or salts or esters or prodrugs thereof, wherein the rhein or diacerein is melt granulated with pharmaceutically acceptable carriers, and wherein the composition exhibits no significant difference in one or both of the rate and the extent of absorption of the rhein or diacerein as compared to a 50 mg diacerein formulation marketed under the trade name Art 50^{®}.

The composition can be taken with or without food.

Embodiments of the pharmaceutical composition may include one or more of the following features. The pharmaceutical composition may further include one or more pharmaceutically acceptable excipients. For example, the pharmaceutically acceptable excipients may include one or more of fillers, binders, lubricants, sweeteners, colors, disintegrants, surfactants, glidants, and the like.

The pharmaceutical composition of the present invention can be present in the form of a tablet, capsule, powder, disc, caplet, granules, pellets, granules in capsule, minitablets, minitablets in capsule, pellets in capsule, sachet and other dosage forms suitable for oral administration. In another general aspect there is provided a process for preparing a pharmaceutical composition of rhein or diacerein, or salts or esters or prodrugs thereof, the process comprising mixing rhein or diacerein, or salts or esters or prodrugs thereof with one or more pharmaceutically acceptable carriers, and granulating the mixture by melting, mixing, congealing, optionally with one or more pharmaceutically acceptable excipients.

In another general aspect there is provided an immediate release wet granulated pharmaceutical composition comprising from about 20 mg to about 45 mg of rhein or diacerein, or salts or esters or prodrugs thereof.

In another general aspect there is provided an immediate release wet granulated pharmaceutical composition comprising from about 20 mg to about 45 mg of rhein or diacerein, or salts or esters or prodrugs thereof, wherein the composition exhibits no significant difference in the rate and the extent of absorption of the rhein or diacerein or salts or esters or prodrugs thereof, as compared to a 50 mg diacerein formulation currently marketed under the trade name Art 50^{®}.

The composition can be taken with or without food.

Embodiments of the pharmaceutical composition may include one or more of the following features. The pharmaceutical composition may further include one or more pharmaceutically acceptable excipients. For example, the pharmaceutically acceptable excipients may include one or more of fillers, binders, lubricants, sweeteners, colors, disintegrants, surfactants, glidants, and the like.

The pharmaceutical composition of the invention can be present in the form of tablet, capsule, powder, disc, caplet, granules, pellets, granules in capsule, minitablets, minitablets in capsule, pellets in capsule, sachet and other dosage forms suitable for oral administration.

In another general aspect there is provided a process for preparing an immediate release pharmaceutical composition comprising from about 20 mg to about 45 mg of rhein or diacerein, or salts or esters or prodrugs thereof, the process comprising:
a) mixing rhein or diacerein, or salts or esters or prodrugs thereof with other pharmaceutically acceptable excipients to form a premix;
b) granulating the premix with one or more suitable solvents; and
c) converting the granules into a suitable dosage form

In another general aspect there is provided a pharmaceutical composition comprising from about 20mg to about 45 mg of rhein or diacerein, or salts or esters or prodrugs thereof, wherein the particles have an average particle size from about 0.1 microns to about 30 microns.

In another general aspect there is provided an oral pharmaceutical composition comprising from about 20mg to about 45 mg of rhein or diacerein, or salts or esters or prodrugs thereof, wherein the particles have an average particle size from about 0.1 microns to about 30 microns, and the composition no significant difference in the rate and the extent of absorption of the rhein or diacerein or salts or esters or prodrugs thereof as compared to that obtained by a 50 mg diacerein formulation marketed under the trade name Art 50^{®}. The composition can be taken with or without food.

Embodiments of the pharmaceutical composition may include one or more of the following features. The pharmaceutical composition may further include one or more pharmaceutically acceptable excipients. For example, the pharmaceutically acceptable excipients may include one or more of fillers, binders, lubricants, sweeteners, glidants, disintegrants, and the like.

The pharmaceutical composition of the invention can be present in the form of tablet, capsule, powder, disc, caplet, granules, beads, sachet, suspension, pellets, spheroids, granules in capsule, minitablets, minitablets in capsule, pellets in capsule, sachet and other dosage forms suitable for oral administration.

In another aspect there is provided a method of making a pharmaceutical composition, the method comprising providing particles of rhein or diacerein, or salts or esters or prodrugs thereof, wherein the particles have an average particle size from about 0.1 microns to about 30 microns; forming a mixture by mixing the particles of rhein or diacerein or salts or esters thereof with one or more pharmaceutically acceptable excipients; and forming the mixture into a pharmaceutical dosage form.

In another general aspect there is provided a modified release pharmaceutical composition comprising rhein or diacerein, or salts or esters or prodrug thereof.

The modified release may be achieved either by one or more functional coatings or mixing the rhein or diacerein, or salts or esters or prodrug thereof with one or more pharmaceutically acceptable polymers.

In another general aspect there is provided a modified release pharmaceutical composition comprising rhein or diacerein, or salts or esters or prodrug thereof, wherein the composition exhibits no significant difference in the rate and the extent of absorption of the rhein or diacerein as compared to 50 mg diacerein formulation marketed under the trade name Art 50^{®}, and wherein the modified release is achieved by one or more functional coatings or mixing the rhein or diacerein, or salts or esters or prodrug thereof with one or more pharmaceutically acceptable polymers.

Embodiments of the pharmaceutical composition may include one or more of the following features. The pharmaceutical composition may further include one or more pharmaceutically acceptable excipients. For example, the pharmaceutically acceptable excipients may include one or more of fillers, binders, lubricants, sweeteners, colors, disintegrants, surfactants, glidants, and the like.

The pharmaceutical composition of the invention can be present in the form of a tablet, capsule, powder, disc, caplet, granules, beads, sachet, suspension, pellets, spheroids, granules in capsule, minitablets, minitablets in capsule, pellets in capsule, sachet and other dosage forms suitable for oral administration.

In another general aspect there is provided a process for preparing a modified release pharmaceutical composition comprising rhein or diacerein, or salts or esters or prodrug thereof, the process comprising coating or mixing rhein or diacerein, or salts or esters or prodrug thereof with one or more pharmaceutically acceptable polymers, optionally with other pharmaceutical excipients and converting the mixture into a suitable dosage form.

In another general aspect there is provided a method of treatment of osteoarthritis, the method comprising providing a pharmaceutical dosage form comprising from about 20mg to about 45mg of rhein or diacerein, or salts or esters or prodrugs thereof to a patient twice daily from day one of therapy.

Embodiments of the pharmaceutical composition may include one or more of the following features. The pharmaceutical composition may further include one or more pharmaceutically acceptable excipients. For example, the pharmaceutically acceptable excipients may include one or more of fillers, binders, lubricants, sweeteners, colors, disintegrants, surfactants, glidants, and the like.

The pharmaceutical composition of the invention can be present in the form of a tablet, capsule, powder, disc, caplet, granules, beads, sachet, suspension, pellets, spheroids, granules in capsule, minitablets, minitablets in capsule, pellets in capsule, sachet and other dosage forms suitable for oral administration.

In another general aspect there is provided an oral pharmaceutical composition comprising from about 20mg to about 45 mg of rhein or diacerein, or salts or esters or prodrugs thereof, wherein the composition exhibits at least about 25% reduction in soft stools as compared to a 50 mg diacerein formulation marketed under the trade name Art 50^{®}.

In another general aspect of the invention there is provided an oral pharmaceutical composition comprising 20 to 45 mg of rhein or diacerein, or salts or esters or prodrugs thereof, wherein one or both of the rate and extent of absorption of the rhein or diacerein is equal or greater than that obtained by a 50 mg diacerein formulation marketed under the trade name Art 50^{®}. The composition exhibits at least 25% reduction in soft stool effect as compared to a 50 mg diacerein formulation marketed under the trade name Art 50^{®}.

Embodiments of the pharmaceutical composition may include one or more of the following features. The pharmaceutical composition may further include one or more pharmaceutically acceptable excipients. For example, the pharmaceutically acceptable excipients may include one or more of fillers, binders, lubricants, sweeteners, colors, disintegrants, surfactants, glidants, and the like.

The pharmaceutical composition of the invention can be present in the form of tablet, capsule, powder, disc, caplet, granules, beads, sachet, suspension, pellets, spheroids, granules in capsule, minitablets, minitablets in capsule, pellets in capsule, sachet and other dosage forms suitable for oral administration.

The details of one or more embodiments of the inventions are set forth in the description below. Other features, objects and advantages of the inventions will be apparent from the description and claims.

### Detailed Description of the Intention

The inventors have developed various techniques for preparing rhein or diacerein compositions which are either bioequivalent or have increased bioavailability than 50mg diacerein composition commercially marketed under the trade name Art 50^{®}.

According to one embodiment, when rhein or diacerein is melt granulated with one or more pharmaceutically acceptable carriers, the obtained melt significantly enhances the solubility of diacerein and percent drug release of diacerein as compared to Art 50^{®}. Art 50^{®} releases about 14% of diacerein in 60 minutes, whereas the pharmaceutical composition of the invention releases 100% of diacerein in 30 minutes. This leads to increased bioavailability. The increased bioavailability further leads to reduction in side effects i.e. soft stools. The administration of the composition to a human subject in a fasted state is bioequivalent to administration of the composition to a subject in fed state in particular, as defined by Cₘₐₓ, Tₘₐₓ and AUC guidelines given by the US Food and Drug Administration (US FDA) and European Medicines Agency (EMEA).

In one embodiment, a pharmaceutical composition of the invention may be prepared by melting one or more pharmaceutically acceptable carriers and mixing the diacerein with the molten mass, followed by congealing. The congealed solid may be sized into granules. The granules may be mixed with other pharmaceutically acceptable excipients and may be formulated into a suitable dosage form. The diacerein may be mixed with the molten mass along with one or more surfactants.

Embodiments of the composition may include one or more of the following features. For example, the pharmaceutical composition may further include one or more pharmaceutically acceptable excipients selected from the group of fillers, lubricants, sweeteners, colors, disintegrants, surfactants and glidants.

Suitable pharmaceutically acceptable carriers include one or more of fatty esters, fatty acids and salts thereof, fatty alcohols, fatty amines, fatty amides, glycerides, glycolipids, steroids, natural and synthetic waxes, polyethylene glycol or its derivatives, and the like.

Polyethylene glycol or its derivatives may include PEG 200, PEG 300, PEG 400, PEG 600, PEG 1000, PEG 4000, PEG 6000, PEG 8000, PEG 20000, polyglycolyzed glycerides, polyethylene glycol-polyoxyethylenes, polyethylene glycol polypropylenes, polyethylene glycolpolyoxypropylenes.

Suitable fatty esters may include triglyceryl ester, glyceryl distearate, glyceryl tristearate, glyceryl monostearate, glyceryl dipalmitate, glyceryl tripalmitate, glyceryl monolaurate, glyceryl dodecosanoate, glyceryl tridecosanoate, glyceryl monodecosanoate, glyceryl monocaprate, glyceryl dicaprate, glyceryl tricaprate, glyceryl monomyristate, glyceryl dimyristate, glyceryl trimyristate, glyceryl monodecanoate, glyceryl didecosanoate, glyceryl tridecosanoate, and the like.

Suitable fatty acids may include acids having 12 to 28 carbons, e.g., stearic acid, palmitic acid, lauric acid, eleostearic acid, etc. Fatty alcohols may comprise compounds having from 16 to 44 carbons, e.g., stearic alcohol, palmitol, etc.

Glycerides may include monoglycerides, diglycerides, triglycerides, glycolipids, steroids and organic salts of fatty acids having from 12 to 29 carbons. Examples thereof comprise stearin, palmitin, castor wax, lecithin, hydrogenated cottonseed oil, hydrogenated tallow, magnesium stearate and calcium and aluminum salts of palmitic and other fatty acids.

Waxes may include paraffin wax, beeswax, carnauba, jojoba, microcrystalline, palm, spermaceti, wool wax and other room temperature-solid hydrocarbon waxes.

Suitable surfactants may include amphoteric, non-ionic, cationic or anionic surfactants. Suitable surfactants comprises one or more of sodium lauryl sulfate, monooleate, monolaurate, monopalmitate, monostearate or another ester of polyoxyethylene sorbitane, sodium dioctylsulfosuccinate (DOSS), lecithin, stearylic alcohol, cetostearylic alcohol, cholesterol, polyoxyethylene ricin oil, polyoxyethylene fatty acid glycerides, poloxamer, cremophore RH 40, and the like.

In yet another embodiment, the inventors have discovered that when rhein or diacerein, or salts or esters or prodrugs thereof is granulated with a suitable solvent or mixture of solvents, it results in a significant increase in the solubility of diacerein and increase in percent drug release of diacerein as compared to Art 50^{®}. The Art 50^{®} prepared by a dry compaction method releases about 14% of diacerein in 60 minutes, whereas the pharmaceutical composition of the present invention prepared by a wet granulation technique releases more than 65% of diacerein in 60 minutes. This results in increase in bioavailability and a significant reduction in side effects i.e. soft stools. The administration of the composition to a human subject in a fasted state is bioequivalent to the administration of the composition to a subject in fed state in particular, as defined by Cₘₐₓ, Tₘₐₓ and AUC guidelines given by the US Food and Drug Administration (US FDA) and European Medicines Agency (EMEA).

According to one embodiment, a pharmaceutical composition can be prepared by mixing diacerein with other pharmaceutically acceptable excipients to form a premix; granulating the pre-mix with one or more suitable solvents; drying the granules; lubricating the granules and converting the final mixture into a suitable dosage form.

Suitable solvents include one or more of water, methanol, ethanol, isopropyl alcohol, acetone, methylene chloride, and the like.

The inventors have also found that when rhein or diacerein, or salts or esters or prodrugs thereof having an average particle size from about 0.1 microns to about 30 microns is used, it results in a significant increase in the solubility of diacerein, thus leading to increase in percent drug release of diacerein as compared to Art 50^{®}. The Art 50^{®} having a particle size of diacerein more than 43 microns releases about 14% of diacerein in 60 minutes, whereas the pharmaceutical composition of the present invention having an average particle size of diacerein from 0.1 microns to 30 microns, in particular, from 0.2 micron to 20 microns releases 100% of diacerein in 15 minutes. The increase in bioavailability results in significant reduction in side effects i.e. soft stools. The administration of the composition to a human subject in a fasted state is bioequivalent to administration of the composition to a subject in fed state in particular as defined by Cₘₐₓ, Tₘₐₓ and AUC guidelines given by US FDA and EMEA.

The inventors have further found that there is no need to co-micronize diacerein with any surfactant to get a formulation, which is bioequivalent to the commercially available diacerein 50 mg solid oral dosage form (Art 50^{®}). Further, the compositions can be administered with or without food. The compositions of the invention result in a significant reduction of soft stools as a side effect, which is commonly encountered with Art 50^{®}.

According to one embodiment, a pharmaceutical composition of the invention may be prepared by dispersing diacerein or salts thereof, optionally with pharmaceutically acceptable excipients in a suitable liquid dispersion medium and wet milling the dispersion through a suitable mill to get a suitable size. The microparticulate dispersion of diacerein or salts thereof may be spray dried in a fluidized bed processor. The dried mixture may be mixed with other pharmaceutically acceptable excipients and may be converted into a suitable dosage form.

It was also found out by the inventors that the wet milling technique results in a better reduction of the particle size of diacerein or rhein. A smaller particle size up to 0.1 microns can be achieved with this technique which is otherwise difficult to achieve with a dry milling technique. Additionally, the wet milling prevents fuming of dust particles and loss resulting from the same.

Suitable liquid dispersion medium includes one or more of water, ethanol, isopropyl alcohol, butanol, hexane, glycols, vegetable oils, mineral oils, and the like.

Suitable means applied to reduce the particle size of rhein or diacerein or salts or esters or prodrugs thereof include one or more of nano mill, ball mill, attrition mill, vibratory mill, sand mill, bead mill, jet mill, dyno mill, ultrasonication, pressure homogenizer microfluidizer, and the like.

The inventors have also observed that when rhein or diacerein is present in an immediate release form, the rhein or diacerein gets immediately absorbed, resulting in a rapid elimination phase and hence decreased area under the plasma time curve (AUC). Additionally, the diacerein is also exposed to harsh gastric conditions leading to degradation of the diacerein. The inventors have overcome this challenge by developing a modified release pharmaceutical composition comprising rhein or diacerein. The modified release pharmaceutical composition results in a prolonged absorption phase and increase in area under the plasma time curve (AUC), thus leading to increased bioavailability. Further, it prevents the exposure of entire diacerein to harsh gastric conditions, hence preventing its degradation. Additionally, the modified release pharmaceutical composition of the invention is bioequivalent to a 50 mg of diacerein formulation commercially marketed under the trade name Art 50^{®}.

The term 'modified release' as used herein includes extended release or delayed release or combinations thereof with immediate release in any percentage weight ratios. The term extended release may be used interchangeably with prolonged release, controlled release, slow release or sustained release.

The modified release composition may be administered in twice daily dosage regimen.

The modified release in the pharmaceutical composition may be achieved by one or more functional coatings or mixing the rhein or diacerein with one or more pharmaceutically acceptable polymers. The release may also be achieved by attachment of rhein or diacerein to ion exchange resins.

The pharmaceutically acceptable polymers may include one or more of rate controlling polymers, or enteric polymers.

Suitable rate controlling polymers may include one or more of polyvinyl acetate, cellulose acetate, cellulose acetate butyrate, cellulose acetate propionate, ethyl cellulose, a fatty acid, a fatty acid ester, an alkyl alcohol, a wax, shellac, rosin, zein (prolamine from corn), a poly(meth)acrylate, microcrystalline cellulose or poly(ethylene oxide), polyuronic acid salts, cellulose ethers, xanthan gum, tragacanth gum, gum karaya, guar gum, acacia, gellan gum locust bean gum, alkali metal salts of alginic acid or pectic acid, sodium alginate, potassium alginate, ammonium alginate, hydroxypropyl cellulose, hydroxypropyl methyl cellulose, carboxyvinyl polymers, and the like.

Suitable enteric polymers may include one or more of polymerized gelatin, shellac, methacrylic acid copolymer type C NF, cellulose butyrate phthalate, cellulose hydrogen phthalate, cellulose propionate phthalate, polyvinyl acetate phthalate (PVAP), cellulose acetate phthalate (CAP), cellulose acetate trimellitate (CAT), hydroxypropyl methylcellulose phthalate, hydroxypropyl methylcellulose acetate, dioxypropyl methylcellulose succinate, carboxymethyl ethylcellulose (CMEC), hydroxypropyl methylcellulose acetate succinate (HPMCAS), and acrylic acid polymers and copolymers like methyl acrylate, ethyl acrylate, methyl methacrylate and/or ethyl methacrylate with copolymers of acrylic and methacrylic acid esters (Eudragit NE, Eudragit RL, Eudragit RS), and the like.

The rhein or diacerein or salts or esters or prodrug thereof may be present in the form of powder, granules, pellets, beads, microtablets, minitablets and crystals.

"Bioequivalency" is established by a 90% Confidence Interval (CI) of between 0.80 and 1.25 for both Cₘₐₓ and AUC under USFDA regulatory guidelines, or a 90% CI for AUC of between 0.80 to 1.25 and a 90% CI for Cₘₐₓ of between 0.70 to 1.43 under the European regulatory guidelines (EMEA).

The term "confidence interval" as used herein refers to the plain meaning known to ordinary skill in the art. The confidence interval refers to a statistical range with a specified probability that a given parameter lies within the range.

The term "covariance" as used herein refers to the plain meaning known to ordinary skill in the art. It is a statistical measure of the variance of two random variables that are observed or measured in the same mean time period. This measure is equal to the product of the deviations of corresponding values of the two variables from their respective means.

The bioequivalence studies were carried out between Art 50^{®} and composition of the invention in both fed state and fasted state. The study was monitored in terms of Cmax, AUC, Tmax achieved with the test products and reference product (Art 50^{®}).

The compositions of the invention exhibits pharmacokinetic profile characterized by Cₘₐₓ of about 3.15 to 6.0µg/ml, Tₘₐₓ of about 2.4 to 5.0h, AUC₀₋ₜ of about 16.4 to 40 µg.h/ml, AUC_{Φ} of about 16.7 to 40 µg.h/ml.

At 90% confidence interval, the area under the concentration time curve (AUC₀₋ₜ and /or AUC_{Φ}) and maximum plasma concentration (Cmax) values of composition of the invention lies between 0.70 and 1.70 as compared to that obtained by a 50 mg diacerein formulation marketed under the trade name Art 50^{®}.

The advantages of the compositions of the invention, include, but are not limited to: (1) smaller solid dosage form size; (2) smaller doses of drug required to obtain the same pharmacological effect; (3) increased bioavailability; (4) substantially similar pharmacokinetic profiles of the rhein or diacerein, compositions when administered in the fed versus the fasted state; (5) bioequivalency of the diacerein compositions when administered in the fed versus the fasted state.

The pharmaceutical compositions may include one or more pharmaceutically acceptable excipients selected from the group of fillers, binders, lubricants, disintegrants, sweeteners, colors, glidants, surfactants, and the like.

Suitable fillers may include one or more of microcrystalline cellulose, silicified microcrystalline cellulose, mannitol, calcium phosphate, calcium sulfate, kaolin, dry starch, powdered sugar, and the like.

Suitable binders may include one or more of povidone, starch, stearic acid, gums, hydroxypropylmethyl cellulose, and the like.

Suitable lubricants may include one or more of magnesium stearate, zinc stearate, calcium stearate, stearic acid, sodium stearyl fumarate, hydrogenated vegetable oil, glyceryl behenate, and the like.

Suitable disintegrants may include one or more of starch, croscarmellose sodium, crospovidone, sodium starch glycolate, and the like.

Suitable glidants may include one or more of colloidal silicon dioxide, talc or cornstarch, and the like.

Suitable sweeteners may include sugars, aspartame, sodium saccharine, potassium acesulfame, neohesperidine, dihydrochalone, sucralose, monoammonium glycyrrhizinate, and mixtures thereof.

The coloring agents of the present invention may be selected from any FDA approved colors for oral use.

The pharmaceutical composition of the present invention can be present in the form of granules, pellets, beads, spheroids, tablet, minitablet, microtablet, capsule, granules in capsule, pellets in capsule, minitablet in capsule or combinations thereof.

The invention is further illustrated by the following examples which are provided merely to be exemplary of the invention and do not limit the scope of the invention. Certain modifications and equivalents will be apparent to those skilled in the art and are intended to be included within the scope of the invention.

### Example 1:

**Table 1**

| **No** | **Ingredients** | **% Composition** |
|---|---|---|
| **Part I** | | |
| 1 | PEG 6000 | 40-60 |

| **Part II** | | |
|---|---|---|
| 2 | Diacerein | 10-60 |
| 4 | Lactose | 5-40 |
| 5 | Croscarmellose sodium | 10-25 |
| 6 | Silicified microcrystalline cellulose | 1-25 |
| 7 | Magnesium stearate | 1-15 |

Procedure: PEG 6000 was melted and mixed at 60-70°C along with diacerein to form a homogenous dispersion followed by congealing while mixing at room temperature. The congealed solid was sized through a sieve to get granules of uniform size. The granules thus obtained were mixed with lactose, croscarmellose sodium, silicified microcrystalline cellulose, lubricated with magnesium stearate and filled into hard gelatin capsules.

### Example 2:

**Table 2**

| **No** | **Ingredients** | **% Composition** |
|---|---|---|
| **Part** I | | |
| 1 | PEG 6000 | 40-60 |
| 2 | Poloxamer | 5-40 |

| **Part II** | | |
|---|---|---|
| 3 | Diacerein | 10-60 |
| 4 | Sodium lauryl sulfate | 1-20 |

| **Part III** | | |
|---|---|---|
| 5 | Silicified microcrystalline cellulose | 1-25 |
| 6 | Lactose | 5-40 |
| 7 | Croscarmellose sodium | 1-40 |
| 8 | Magnesium stearate | 1-15 |

Procedure: Poloxamer and PEG 6000 were melted and mixed at 60-70°C along with diacerein, sodium lauryl sulfate to form a homogenous dispersion followed by congealing while mixing at room temperature. The congealed solid was sized through a sieve to get granules of uniform size. The granules thus obtained were mixed with lactose, silicified microcrystalline cellulose, croscarmellose sodium and lubricated with magnesium stearate and filled into hard gelatin capsules.

**Table 3: Dissolution Data**

| **Time (min)** | **% drug released (Art 50^{®})** | **% drug released (Example-2)** |
|---|---|---|
| **5** | 3 | 38 |
| **10** | 4 | 74 |
| **15** | 5 | 88 |
| **20** | 7 | 97 |
| **30** | 9 | 100 |
| **45** | 11 | 100 |
| **60** | 14 | 100 |

For determination of drug release rate, USP Type 2 Apparatus (rpm 75) was used wherein 1000 ml of pH 5.7 phosphate buffer at 37 °C ± 0.5°C was used as a medium.

### Example 3:

**Table 4**

| **S. No** | **Ingredients** | **Quantity (mg per tablet)** |
|---|---|---|
| **Intragranular** | | |
| 1 | Diacerein | 44.00 |
| 2 | Povidone | 11.50 |
| 3 | Lactose | 220.30 |
| 4 | Croscarmellose sodium | 11.50 |
| 5 | Colloidal silicon dioxide | 5.75 |
| 6 | Hydroxy propyl methyl cellulose | 20.00 |

| **Extragranular** | | |
|---|---|---|
| 7 | Colloidal silicon dioxide | 5.75 |
| 8 | Magnesium stearate | 1.20 |

Procedure: Diacerein was mixed with lactose, croscarmellose sodium, colloidal silicon dioxide, hydroxypropylmethyl cellulose and granulated with aqueous solution of povidone. The granules were dried, mixed with colloidal silicon dioxide and lubricated with magnesium stearate and filled into hard gelatin capsules of a suitable size.

**Table 5: Dissolution data**

| **Time (min)** | **% drug released (Art 50^{®})** | **% drug released (Example-3)** |
|---|---|---|
| **10** | 4 | 17 |
| **20** | 7 | 28 |
| **30** | 9 | 39 |
| **45** | 11 | 54 |
| **60** | 14 | 68 |
| **90** | 16 | 94 |
| **120** | 19 | 100 |

Table 5 provides the dissolution data for diacerein capsules prepared as per the formula given in Table 4. For determination of drug release rate, USP Type 2 Apparatus (rpm 75) was used wherein 1000 ml of pH 5.7 phosphate buffer at 37 °C ± 0.5°C was used as a medium.

### Example 4:

**Table 6**

| **S. No** | **Ingredients** | **Quantity (mg per tablet)** |
|---|---|---|
| **Intragranular** | | |
| 1 | Diacerein | 45.00 |
| 2 | Povidone | 11.50 |
| 3 | Lactose | 219.30 |
| 4 | Croscarmellose sodium | 11.50 |
| 5 | Colloidal silicon dioxide | 5.75 |
| 6 | Hydroxy propyl methyl cellulose | 20.00 |

| **Extragranular** | | |
|---|---|---|
| 7 | Colloidal silicon dioxide | 5.75 |
| 8 | Magnesium stearate | 1.20 |

Procedure: Diacerein was mixed with lactose, croscarmellose sodium, colloidal silicon dioxide, hydroxypropylmethyl cellulose and granulated with aqueous solution of povidone. The granules were dried, mixed with colloidal silicon dioxide and lubricated with magnesium stearate and filled into hard gelatin capsules of a suitable size.

**Table 7: Dissolution data**

| **Time (min)** | **% drug released (Art 50^{®})** | **% drug released (Example-4)** |
|---|---|---|
| **10** | 4 | 18 |
| **20** | 7 | 26 |
| **30** | 9 | 37 |
| **45** | 11 | 57 |
| **60** | 14 | 72 |
| **90** | 16 | 96 |
| **120** | 19 | 100 |

Table 7 provides the dissolution data for diacerein capsules prepared as per the formula given in Table 6. For determination of drug release rate, USP Type 2 Apparatus (rpm 75) was used wherein 1000 ml of pH 5.7 phosphate buffer at 37 °C ± 0.5°C was used as a medium.

**Table 8: Bioequivalence data of the composition of the invention against Art 50^{®} with respect to pharmacokinetic parameters**

| **S.N.** | **Pharmacokinetic parameters** | **(Art 50^{®})** | **Composition of the invention** |
|---|---|---|---|
| 1 | Cₘₐₓ (µg/ml) | 3.058 | 3.48 |
| 2 | Tₘₐₓ (h) | 5.39 | 4.80 |
| 3 | AUC₀₋ₜ (µgh/ml) | 22.688 | 22.82 |
| 4 | AUC_{Φ}(µgh/ml) | 22.816 | 23.62 |

**Table-9: Bioequivalence data with respect to Test (Composition of the invention) to reference (Art 50^{®}) Ratios (T/R ratios) at 90% Confidence Interval (C.I.)**

| **S. No** | **Pharmacokinetic parameters** | **Ratio** | **90% C.I.** | | **% CV** |
|---|---|---|---|---|---|
| | | | **Lower** | **Upper** | |
| 1 | Cₘₐₓ (µg/ml) | 113.79 | 97.56 | 179.09 | 25.23 |
| 2 | AUG₀₋ₜ (µgh/ml) | 100.57 | 89.48 | 150.17 | 22.39 |
| 3 | AUC_{Φ}(µgh/ml) | 103.51 | 85.26 | 148.29 | 23.79 |

### Example 5:

**Table 10**

| **S.N.** | **Ingredients** | **%w/w** |
|---|---|---|
| | **Part-I** | |
| 1 | Diacerein | 11.26 |
| 2 | Povidone | 5-40 |
| 3 | Water | q.s. |

| | **Part-II** | |
|---|---|---|
| 4 | Starch | 10-50 |
| 5 | Silicified microcrystalline cellulose | 5-70 |
| 6 | Croscarmellose sodium | 1-15 |
| 7 | Magnesium stearate | 0.1-3 |

Procedure: Diacerein and povidone was dispersed in sufficient quantity of water to get a uniform dispersion. The diacerein dispersion was passed through a pressure homogenizer one or more times to get a desired particle size (0.1µ). The microparticulate dispersion was spray dried in glatt. The dried mass was blended with silicified microcrystalline cellulose, starch, croscarmellose sodium, lubricated with magnesium stearate and lubricated blend was filled into hard gelatin capsules of a suitable size.

### Example 6:

**Table 11**

| **S.N.** | **Ingredients** | **%w/w** |
|---|---|---|
| | **Part-I** | |
| 1 | Diacerein | 11.26 |
| 2 | Povidone | 5-40 |
| 3 | Water | q.s. |

| | **Part-II** | |
|---|---|---|
| 4 | Lactose | 10-50 |
| 5 | Silicified microcrystalline cellulose | 5-70 |
| 6 | Croscarmellose sodium | 1-15 |
| 7 | Magnesium stearate | 0.1-3 |

Procedure: Diacerein and povidone was dispersed in sufficient quantity of water to get a uniform dispersion. The diacerein dispersion was passed through a microfluidizer one or more times to get a desired particle size (0.8µ). The microparticulate dispersion was spray dried in glatt. The dried mass was blended with silicified microcrystalline cellulose, lactose, croscarmellose sodium, lubricated with magnesium stearate and lubricated blend was filled into hard gelatin capsules of a suitable size.

### Example 7:

**Table 12**

| **S.N.** | **Ingredients** | **%w/w** |
|---|---|---|
| | **Part-I** | |
| 1 | Diacerein | 9.50 |
| 2 | Hydroxypropyl methyl cellulose | 10-50 |
| 3 | Water | q.s. |

| | **Part-II** | |
|---|---|---|
| 4 | Povidone | 5-40 |
| 5 | Silicified microcrystalline cellulose | 5-70 |
| 6 | Croscarmellose sodium | 1-15 |
| 7 | Magnesium stearate | 0.1-3 |

Procedure: Diacerein and povidone was dispersed in sufficient quantity of water to get a uniform dispersion. The diacerein dispersion was passed through a pressure homogenizer one or more times to get a desired particle size (5µ). The microparticulate dispersion was spray dried in glatt. The dried mass was blended with silicified microcrystalline cellulose, microcrystalline cellulose, croscarmellose sodium, lubricated with magnesium stearate and lubricated blend was filled into hard gelatin capsules of a suitable size.

### Example 8:

**Table 13**

| **S.N.** | **Ingredients** | **%w/w** |
|---|---|---|
| | **Part-I** | |
| 1 | Diacerein | 9.50 |
| 2 | Microcrystalline cellulose | 5-40 |
| 3 | Water | q.s. |

| | **Part-II** | |
|---|---|---|
| 4 | Starch | 10-50 |
| 5 | Silicified microcrystalline cellulose | 5-70 |
| 6 | Croscarmellose sodium | 1-15 |
| 7 | Magnesium stearate | 0.1-3 |

Procedure: Diacerein and hydroxypropylmethyl cellulose was dispersed in sufficient quantity of water to get a uniform dispersion. The diacerein dispersion was passed through a pressure homogenizer one or more times to get a desired particle size (12µ). The microparticulate dispersion was spray dried in glatt. The dried mass was blended with silicified microcrystalline cellulose, starch, croscarmellose sodium, lubricated with magnesium stearate and lubricated blend was filled into hard gelatin capsules of a suitable size.

### Example 9:

**Table 14**

| **S.N.** | **Ingredients** | **%w/w** |
|---|---|---|
| | **Part-I** | |
| 1 | Diacerein | 11.80 |
| 2 | Povidone | 5-40 |
| 3 | Water | q.s. |

| | **Part-II** | |
|---|---|---|
| 4 | Sucrose | 10-50 |
| 5 | Starch | 10-50 |
| 6 | Silicified microcrystalline cellulose | 5-70 |
| 7 | Croscarmellose sodium | 1-15 |
| 8 | Magnesium stearate | 0.1-3 |

Procedure: Diacerein and hydroxypropylmethyl cellulose was dispersed in sufficient quantity of water to get a uniform dispersion. The diacerein dispersion was passed through a pressure homogenizer one or more times to get a desired particle size (15µ). The microparticulate dispersion was spray dried in glatt. The dried mass was blended with sucrose, silicified microcrystalline cellulose, starch, croscarmellose sodium, lubricated with magnesium stearate and lubricated blend was filled into hard gelatin capsules of a suitable size.

### Example 10:

**Table 15**

| **S.N.** | **Ingredients** | **%w/w** |
|---|---|---|
| | **Part-I** | |
| 1 | Diacerein | 12.50 |
| 2 | Hydroxypropyl methyl cellulose | 5-40 |
| 3 | Water | q.s. |

| | **Part-II** | |
|---|---|---|
| 4 | Sucrose | 10-50 |
| 5 | Starch | 10-50 |
| 6 | Silicified microcrystalline cellulose | 5-70 |
| 7 | Croscarmellose sodium | 1-15 |
| 8 | Magnesium stearate | 0.1-3 |

Procedure: Diacerein and povidone was dispersed in sufficient quantity of water to get a uniform dispersion. The diacerein dispersion was passed through a microfluidizer one or more times to get a desired particle size (22µ). The microparticulate dispersion was spray dried in glatt. The dried mass was blended with sucrose, silicified microcrystalline cellulose, starch, croscarmellose sodium, lubricated with magnesium stearate and lubricated blend was filled into hard gelatin capsules of a suitable size.

**Table 16: Dissolution data**

| **Time (min)** | **% Drug released (Art 50^{®} with particle size more than 43µ)** | **% Drug released (Composition of the invention with particle size from 0.1-30µ)** |
|---|---|---|
| **5** | 3 | 55 |
| **10** | 4 | 93 |
| **15** | 5 | 100 |
| **20** | 7 | 100 |
| **30** | 9 | 100 |
| **45** | 11 | 100 |
| **60** | 14 | 100 |

For determination of drug release rate, USP Type 2 Apparatus (rpm 75) was used wherein 1000 ml of pH 5.7 phosphate buffer at 37 °C ± 0.5°C was used as medium.

**Table-17: Bioequivalence data of the composition of the invention against Art 50^{®} with respect to pharmacokinetic parameters**

| **S.N.** | **Pharmacokinetic parameters** | **Art 50^{®}** | **Composition of the invention** |
|---|---|---|---|
| 1 | Cₘₐₓ (µg/ml) | 3.058 | 4.050 |
| 2 | Tₘₐₓ (h) | 5.39 | 4.47 |
| 3 | AUC₀₋ₜ (µgh/ml) | 22.688 | 25.559 |
| 4 | AUC_{Φ}(µgh/ml) | 22.816 | 25.675 |

**Table 18: Bioequivalence data with respect to Test (Composition of the invention) to reference (Art 50^{®}) Ratios (T/R ratios) at 90% Confidence Interval (C.I.)**

| **S.No** | **Pharmacokinetic parameters** | **Ratio** | **90% C.I.** | | **% CV** |
|---|---|---|---|---|---|
| | | | **Lower** | **Upper** | |
| 1 | Cₘₐₓ (µg/ml) | 132.42 | 99.59 | 176.08 | 27.73 |
| 2 | AUC₀₋ₜ (µgh/ml) | 112.65 | 87.42 | 145.17 | 24.59 |
| 3 | AUC_{Φ}(µgh/ml) | 112.53 | 87.16 | 145.29 | 24.77 |

### Example 11:

**Table 19**

| **S.N.** | **Ingredients** | **Mg per tablet** |
|---|---|---|
| | **Part-I** | |
| 1 | Diacerein | 44.00 |
| 2 | Povidone | 50.00 |
| 3 | Docusate sodium | 7.00 |
| 4 | Sodium lauryl sulfate | 8.00 |
| 5 | Starch | 160.00 |
| 6 | Silicified microcrystalline cellulose | 30.00 |
| 7 | Water | q.s. |

| | **Part-II** | |
|---|---|---|
| 8 | Silicified microcrystalline cellulose | 79.00 |
| 9 | Croscarmellose sodium | 20.00 |
| 10 | Magnesium stearate | 2.00 |

Procedure: Povidone was dissolved in water to obtain a clear solution. Diacerein was dispersed in the above mentioned povidone solution to form a uniform dispersion. The diacerein dispersion was passed through a pressure homogenizer one or more times to get a desired particle size (0.9µ). Sodium docusate was dissolved in water to obtain a clear solution and this clear solution was added to the microparticulate dispersion of diacerein and the dispersion was spray dried in glatt on starch and silicified microcrystalline cellulose. The dried mass was blended with silicified microcrystalline cellulose, croscarmellose sodium, lubricated with magnesium stearate and lubricated blend was filled into hard gelatin capsules of a suitable size.

**Table 20: Dissolution data**

| **Time (min)** | **% Drug released (Art 50^{®} with particle size more than 43µ)** | **% Drug released (Example 11)** |
|---|---|---|
| **10** | 4 | 93 |
| **20** | 7 | 99 |
| **30** | 9 | 100 |
| **45** | 11 | 100 |
| **60** | 14 | 100 |

For determination of drug release rate, USP Type 2 Apparatus (rpm 75) was used wherein 1000 ml of pH 5.7 phosphate buffer at 37 °C ± 0.5°C was used as medium.

**Table-21: Bioequivalence data of the composition of the invention against Art 50^{®} with respect to pharmacokinetic parameters**

| **S.No** | **Pharmacokinetic parameters** | **Art 50^{®}** | **Composition of the invention** |
|---|---|---|---|
| 1 | Cₘₐₓ (µg/ml) | 3.058 | 4.250 |
| 2 | Tₘₐₓ (h) | 5.39 | 4.17 |
| 3 | AUC₀₋ₜ (µgh/ml) | 22.688 | 25.659 |
| 4 | AUC_{Φ}(µgh/ml) | 22.816 | 25.778 |

**Table-22: Bioequivalence data with respect to Test (Composition of the invention) to reference (Art 50^{®}) Ratios (T/R ratios) at 90% Confidence Interval (C.I.)**

| **S.No** | **Pharmacokinetic parameters** | **Ratio** | **90% C.I.** | | **%CV** |
|---|---|---|---|---|---|
| | | | **Lower** | **Upper** | |
| 1 | Cₘₐₓ (µg/ml) | 139.98 | 97.56 | 179.09 | 25.23 |
| 2 | AUC₀₋ₜ (µgh/ml) | 113.09 | 89.48 | 150.17 | 22.39 |
| 3 | AUC_{Φ}(µgh/ml) | 112.98 | 85.26 | 148.29 | 23.79 |

### Example 12:

**Table 23**

| **Ingredients** | **% w/w** |
|---|---|
| **Part-I** | |
| Diacerein | 5-13 |
| Lactose | 10-50 |
| Povidone | 5-40 |
| Docusate sodium | 1-20 |
| TPGS | 5-30 |
| Starch | 10-50 |
| Prosolv | 5-70 |

| **Part-II** | |
|---|---|
| Dried Granules from Part I | |
| Eudragit | 7.0 |

| **Part III** | |
|---|---|
| Extra granular | |
| Prosolv | 5-70 |
| Ac-Di-Sol | 1-15 |
| Magnesium Stearate | 0.1-3 |

Procedure: Docusate sodium was dissolved in purified water under stirring. Povidone was added under stirring to above solution of sodium docusate to form a clear solution and further diacerein was added to form a uniform dispersion. The diacerein dispersion was passed through Dyno mill/Pressure homogenizer/ microfluidizer to get a suitable particle size. A clear solution of TPGS was added to above microparticulate/ nanoparticulate dispersion of diacerein. The diacerein dispersion was sprayed on starch and prosolv by top spray method on glatt. The dried granules thus obtained were coated with Eudragit and further dried and mixed with prosolv, croscarmellose sodium and lubricated with magnesium stearate. The lubricated blend was filled in suitable size capsules.

**Table 24: Dissolution data**

| **Time (min)** | **% Drug released (Example 12)** |
|---|---|
| **5** | 26 |
| **10** | 44 |
| **15** | 75 |
| **30** | 97 |
| **45** | 98 |
| **60** | 98 |

Table 24 provides the dissolution data for diacerein capsules prepared as per the formula given in Table 23. For determination of drug release rate, USP Type 2 Apparatus (rpm 75) was used wherein 1000 ml of pH 5.7 phosphate buffer at 37 °C ± 0.5°C was used as a medium.

### Example 13:

**Table 25**

| **Ingredients** | **%w/w** |
|---|---|
| **Part-I** | |
| Diacerein | 5-13 |
| Povidone | 5-40 |
| Docusate sodium | 1-20 |
| TPGS | 5-30 |
| Starch | 10-50 |
| Prosolv | 5-70 |

| **Part-II** | |
|---|---|
| Dried Granules from Part I | |
| Eudragit | 4.0 |

| **Part III** | |
|---|---|
| Extra granular | |
| Prosolv | 5-70 |
| Ac-Di-Sol | 1-15 |
| Magnesium stearate | 0.1-3 |

Procedure: Docusate sodium was dissolved in purified water under stirring. PVP was added under stirring to the above solution of sodium docusate to form a clear solution and further diacerein was added to form a uniform dispersion. The diacerein dispersion was passed through Dyno mill/Pressure homogenizer/microfluidizer to get a suitable particle size. A clear solution of TPGS was added to above microparticulate/nanoparticulate dispersion of diacerein. The diacerein dispersion was sprayed on starch and prosolv by top spray method on glatt. The dried granules thus obtained were coated with Eudragit and further dried and mixed with prosolv, croscarmellose sodium and lubricated with magnesium stearate. The lubricated blend was filled in suitable size capsules.

**Table 26: Dissolution data**

| **Time (min)** | **% Drug released (Example-13)** |
|---|---|
| **5** | 32 |
| **10** | 56 |
| **15** | 75 |
| **30** | 85 |
| **45** | 90 |
| **60** | 94 |

Table 26 provides the dissolution data for diacerein capsules prepared as per the formula given in Table 25. For determination of drug release rate, USP Type 2 Apparatus (rpm 75) was used wherein 1000 ml of pH 5.7 phosphate buffer at 37 °C ± 0.5°C was used as medium.

### Example 14:

**Table 27**

| **Ingredients** | **%w/w** |
|---|---|
| **Part-I** | |
| Diacerein | 5-13 |
| Povidone | 5-40 |
| Docusate sodium | 1-20 |
| TPGS | 5-30 |
| Sodium lauryl sulfate | 1-20 |
| Starch | 10-50 |
| Prosolv | 5-70 |

| **Part-II** | |
|---|---|
| Dried Granules from Part I | |
| Eudragit | 4.0 |

| **Part III** | |
|---|---|
| Extra granular | |
| Prosolv SMCC 90 | 5-70 |
| Ac-Di-Sol | 1-15 |
| Magnesium Stearate | 0.1-3 |

Procedure: Docusate sodium was dissolved in purified water under stirring. PVP was added under stirring to the above solution of docusate sodium to form a clear solution and further diacerein was added to form a uniform dispersion. The diacerein dispersion was passed through Dyno mill/Pressure homogenizer/microfluidizer to get a suitable particle size. A clear solution of TPGS & sodium lauryl sulfate was added to the above microparticulate/nanoparticulate dispersion of diacerein. The diacerein dispersion was sprayed on starch and prosolv by top spray method on glatt. The dried granules thus obtained were coated with Eudragit and further dried and mixed with prosolv, croscarmellose sodium and lubricated with magnesium stearate. The lubricated blend was filled in suitable size capsules.

**Table 28: Bioequivalence data of compositions of invention against Art 50^{®} with respect to pharmacokinetic parameters.**

| **S. N.** | **Pharmacokinetic parameters.** | **Art 50^{®}** | **Composition of the invention** |
|---|---|---|---|
| 1 | Cₘₐₓ (µg/ml) | 3.058 | 5.121 |
| 2 | Tₘₐₓ (h) | 5.39 | 5.70 |
| 3 | AUC₀₋ₜ (µg.h/ml) | 22.688 | 24.927 |
| 4 | AUC_{0-inf} (µg.h/ml) | 22.816 | 25.569 |

**Table 29: Bioequivalence data with respect to test (composition of the examples) to reference (Art 50^{®}) ratios (T/R ratios) at 90 % confidence interval.**

| **S. N.** | **Pharmacokinetic parameters** | **Ratio** | **90 % C. I.** | | **% CV** |
|---|---|---|---|---|---|
| | | | **Lower** | **Upper** | |
| 1 | Cₘₐₓ | 120.17 | 107.61 | 134.2 | 13.33 |
| 2 | AUC₀₋ₜ | 94.5 | 91.56 | 97.3 | 3.80 |
| 3 | AUC_{0-inf} | 94.55 | 91.56 | 97.53 | 3.86 |

### Example 15:

**Table 30**

| **Ingredients** | **%w/w** |
|---|---|
| **Part-I** | |
| Diacerein | 5-13 |
| Lactose | 10-50 |
| Povidone | 5-40 |
| Docusate sodium | 1-20 |
| TPGS | 5-30 |
| Starch | 10-50 |
| Prosolv | 5-70 |

| **Part-A (Immediate release portion)** | |
|---|---|
| Dried Granules (Portion containing 65-85% w/w of total diacerein of part I) | |
| **Part-B (Controlled release portion)** | |
| Dried Granules (Portion containing 15%-35% w/w of total diacerein of part I) | |
| Hydroxypropyl methyl cellulose | 3.75 |
| Prosolv | 5-70 |
| Magnesium Stearate | 0.1-3 |

| Extra granular | |
|---|---|
| Part A granules | |
| Part B granules | |
| Prosolv | 5-70 |
| Ac-Di-Sol | 1-15 |
| Magnesium Stearate | 0.1-3 |

Procedure: Docusate sodium was dissolved in purified water under stirring. PVP was added under stirring to the above solution of sodium docusate to form a clear solution and further diacerein was added to form a uniform dispersion. The diacerein dispersion was passed through Dyno mill/Pressure homogenizer/microfluidizer to get a suitable particle size. A clear solution of TPGS was added to the above microparticulate/nanoparticulate dispersion of diacerein. The diacerein dispersion was sprayed on starch and prosolv by top spray method on glatt. The dried granules thus obtained were divided into two parts. First part (A) kept as such as an immediate release portion. To second part, hydroxypropylmethyl cellulose was added along with prosolv, mixed and lubricated with magnesium stearate. The lubricated granules were compacted through a roll compactor and the compacts were granulated through a multimill to get granules of uniform size (Part B). Part B granules were mixed with part A granules along with prosolv, ac-di-sol and magnesium stearate and the final blend was filled in suitable size capsules.

**Table 31: Dissolution data**

| **Time (min)** | **% Drug released (Example-15)** |
|---|---|
| **10** | 45 |
| **20** | 76 |
| **30** | 99 |
| **45** | 100 |
| **60** | 100 |
| **90** | 100 |
| **120** | 100 |

Table 31 provides the dissolution data for diacerein capsules prepared as per the formula given in Table 30. For determination of drug release rate, USP Type 2 Apparatus (rpm 75) was used wherein 1000 ml of pH 5.7 phosphate buffer at 37 °C ± 0.5°C was used as medium.

### Example 16:

**Table 32**

| **Ingredients** | **%w/w** |
|---|---|
| **Part-I** | |
| Diacerein | 5-13 |
| Lactose | 10-50 |
| Povidone | 5-40 |
| Docusate sodium | 1-20 |
| TPGS | 5-30 |
| Starch | 10-50 |
| Prosolv | 5-70 |

| **Part-A (Immediate release portion)** | |
|---|---|
| Dried Granules (Portion containing 65-85% w/w of total diacerein of part I) | |
| **Part-B (Controlled release portion)** | |
| Dried Granules (Portion containing 15%-35% w/w of total diacerein of part I) | |
| Hydroxypropyl methyl cellulose | 7.5 |
| Prosolv | 5-70 |
| Magnesium Stearate | 0.1-3 |

| Extra granular | |
|---|---|
| Part A granules | |
| Part B granules | |
| Prosolv | 5-70 |
| Ac-Di-Sol | 1-15 |
| Magnesium Stearate | 0.1-3 |

Procedure: Docusate sodium was dissolved in purified water under stirring. PVP was added under stirring to the above solution of sodium docusate to form a clear solution and further diacerein was added to form a uniform dispersion. The diacerein dispersion was passed through Dyno mill/Pressure homogenizer/microfluidizer to get a suitable particle size. A clear solution of TPGS was added to the above microparticulate/nanoparticulate dispersion of diacerein. The diacerein dispersion was sprayed on starch and prosolv by top spray method on glatt. The dried granules thus obtained were divided into two parts. First part was (A) kept as such as an immediate release portion. To second part, hydroxypropylmethyl cellulose was added along with prosolv, mixed and lubricated with magnesium stearate. The lubricated granules were compacted through a roll compactor and the compacts were granulated through a multimill to get granules of uniform size (Part B). Part B granules were mixed with part A granules along with prosolv, ac-di-sol and magnesium stearate and the final blend was filled in suitable size capsules.

**Table 33: Dissolution data**

| **Time (min)** | **% Drug released (Example 16)** |
|---|---|
| **10** | 11 |
| **20** | 24 |
| **30** | 36 |
| **45** | 51 |
| **60** | 72 |
| **90** | 92 |
| **120** | 95 |

Table 33 provides the dissolution data for diacerein capsules prepared as per the formula given in Table 32. For determination of drug release rate, USP Type 2 Apparatus (rpm 75) was used wherein 1000 ml of pH 5.7 phosphate buffer at 37 °C ± 0.5°C was used as medium.

### Example 17:

**Table-34**

| **Ingredients** | **%w/w** |
|---|---|
| **Part-I** | |
| Diacerein | 5-13 |
| Lactose | 10-50 |
| Povidone | 5-40 |
| Docusate sodium | 1-20 |
| TPGS | 5-30 |
| Starch | 10-50 |
| Prosolv | 5-70 |

| **Part-A (Immediate release portion)** | |
|---|---|
| Dried Granules (Portion containing 65-85% w/w of total diacerein of part I) | |
| **Part-B (Controlled release portion)** | |
| Dried Granules (Portion containing 15%-35% w/w of total diacerein of part I) | |
| Hydroxypropyl methyl cellulose | 6.25 |
| Prosolv | 5-70 |
| Magnesium Stearate | 0.1-3 |

| **Part-IV (Extra granular)** | |
|---|---|
| Part II granules | |
| Part III granules | |
| Prosolv | 5-70 |
| Ac-Di-Sol | 1-15 |
| Magnesium Stearate | 0.1-3 |

Procedure: Docusate sodium was dissolved in purified water under stirring. PVP was added under stirring to the above solution of sodium docusate to form a clear solution and further diacerein was added to form a uniform dispersion. The diacerein dispersion was passed through Dyno mill/Pressure homogenizer/microfluidizer one or more times to get a suitable particle size. A clear solution of TPGS was added to the above microparticulate/nanoparticulate dispersion of diacerein. The diacerein dispersion was sprayed on starch and prosolv by top spray method on glatt. The dried granules thus obtained were divided into two parts. First part (A) was kept as such as an immediate release portion. To second part, hydroxypropylmethyl cellulose was added along with prosolv, mixed and lubricated with magnesium stearate. The lubricated granules were compacted through a roll compactor and the compacts were granulated through a multimill to get granules of uniform size (Part B). Part B granules were mixed with part A granules along with prosolv, ac-di-sol and magnesium stearate and the final blend was filled in suitable size capsules.

**Table 35: Dissolution data**

| **Time (min)** | **% Drug released (Example-17)** |
|---|---|
| **10** | 24 |
| **20** | 43 |
| **30** | 62 |
| **45** | 80 |
| **60** | 90 |
| **90** | 100 |
| **120** | 100 |

Table 35 provides the dissolution data for diacerein capsules prepared as per the formula given in Table 34. For determination of drug release rate, USP Type 2 Apparatus (rpm 75) was used wherein 1000 ml of pH 5.7 phosphate buffer at 37 °C ± 0.5°C was used as a medium.

**Table 36: Bioequivalence data of composition of the invention against Art 50^{®} with respect to pharmacokinetic parameters.**

| **S. N.** | **Pharmacokinetic parameters** | **Art 50^{®}** | **Composition of the invention** |
|---|---|---|---|
| 1 | Cₘₐₓ (µg/ml) | 3.058 | 4.773 |
| 2 | Tₘₐₓ (h) | 5.39 | 4.44 |
| 3 | AUC₀₋ₜ (µg.h/ml) | 22.688 | 21.651 |
| 4 | AUC_{0-inf} (µg.h/ml) | 22.816 | 22.192 |

**Table 37: Bioequivalence data with respect to test (composition of the invention) to reference (Art 50^{®}) ratios (T/R ratios) at 90 % confidence interval.**

| **S. N.** | **Pharmacokinetic parameters** | **Ratio** | **90 % C. I.** | | **% CV** |
|---|---|---|---|---|---|
| | | | **Lower** | **Upper** | |
| 1 | Cₘₐₓ | 122.63 | 110.76 | 135.78 | 11.15 |
| 2 | AUC₀₋ₜ | 94.89 | 89.71 | 100.36 | 6.13 |
| 3 | AUC_{0-inf} | 94.73 | 89.34 | 100.45 | 6.41 |

While the invention has been described in terms of its specific embodiments, certain modifications and equivalents will be apparent to those skilled in the art and are intended to be included within the scope of the invention.

## Claims

1. A pharmaceutical composition comprising particles of rhein or diacerein or salts or esters or prodrugs thereof, wherein the particles have an average particle size from about 0.1 microns to about 30 microns.

2. A pharmaceutical composition comprising from about 20mg to about 45mg of rhein or diacerein, or salts or esters or prodrugs thereof, wherein the particles have an average particle size from about 0.1 microns to about 30 microns, and optionally one or more pharmaceutically acceptable excipients.

3. The composition of claim 2, wherein the composition exhibits no significant difference in one or both of the rate and the extent of absorption of the rhein or diacerein or salts or esters or prodrugs thereof as compared to that obtained by a 50 mg diacerein formulation marketed under the trade name Art 50®.

4. The composition of claim 2, wherein the composition exhibits a maximum plasma concentration (Cₘₐₓ) from about 3.15 to about 6.0 µg/ml.

5. The composition of claim 2, wherein the composition exhibits a time to reach maximum plasma concentration (Tₘₐₓ) from about 2.2 to about 5.0 h.

6. The composition of claim 2, wherein the composition exhibits an area under the concentration time curve (AUC₀₋ₜ) from about 16.4 to about 40 µg.h/ml.

7. The composition of claim 2, wherein the composition can be taken with or without food.

8. The composition of claim 2, wherein the composition exhibits a dissolution profile such that more than 85% of rhein or diacerein, or salts thereof is released within 20 minutes, wherein the release rate is measured in Apparatus 2 (USP, Dissolution, paddle, 75 rpm) using 1000 ml of pH 5.7 phosphate buffer at 37°C ± 0.5°C.

9. The composition of claim 2, wherein the composition comprises one or more of a tablet, capsule, powder, disc, caplet, granules, pellets, granules in capsule, minitablets, minitablets in capsule, pellets in capsule, sachet, beads, spheroids, suspension and tablet in tablet.

10. The composition of claim 2, wherein the composition comprises about 44 mg of rhein or diacerein or salts or esters or prodrugs thereof.

11. A method of making a pharmaceutical composition, the method comprising providing particles of rhein or diacerein, or salts or esters or prodrugs thereof, wherein the particles have an average particle size from about 0.1 microns to about 30 microns; forming a mixture by mixing the particles of rhein or diacerein or salts or esters thereof with one or more pharmaceutically acceptable excipients; and forming the mixture into a pharmaceutical dosage form.

12. The method of claim 11, wherein providing the particles comprises dispersing rhein or diacerein, or salts or esters or prodrugs thereof in a suitable liquid dispersion medium and reducing the particle size of the rhein or diacerein, or salts or esters or prodrugs thereof.

13. The method of claim 12, wherein the suitable liquid dispersion medium comprises one or more of water, ethanol, isopropyl alcohol, butanol, hexane, glycols, vegetable oils, and mineral oils.

14. The method of claim 12, wherein the particle size reduction is carried out by a wet milling technique.
